# EUROPEAN PATENT SPECIFICATION

(11) **EP 1 794 287 B1**
(45) Date of publication and mention of the grant of the patent: **22.07.2009**
(21) Application number: 05806042.7
(22) Date of filing: 30.09.2005
(51) Int. Cl.: C12N 5/06, C12N 5/08, C07K 14/54, A61K 38/20

(54) **USE OF IL-17- FOR MATURATION OF OOCYTES**
VERWENDUNG VON INTERLEUKIN-17 FÜR OOZYTENREIFUNG
UTILISATION D'IL-17 POUR LA MATURATION D'OVOCYTES

(30) Priority: 30.09.2004 US 614667 P
(43) Date of publication of application: 13.06.2007
(73) Proprietor: Merck Serono SA, 1267 Coinsins, Vaud (CH)
(72) Inventor: DE MATOS, Daniel, G., Pembroke, MA 02359 (US); TRAN, Cam Anh, Quincy, MA 02170 (US); CLARK, Ann, M., Weymouth, MA 02189 (US); PALMER, Stephen, S., Plympton, MA 02367 (US)
(74) Representative: Sutcliffe, Nicholas Robert
(86) International application number: PCT/US2005/035372
(87) International publication number: WO 2006/039592

(56) References cited:
- EP-A- 1 215 280
- WO-A-03/076600
- US-A1- 2003 124 092
- GOUD P T ET AL: "IN-VITRO MATURATION OF HUMAN GERMINAL VESICLE STAGE OOCYTES: ROLE OF CUMULUS CELS AND EPIDERMAL GROWTH FACTOR IN THE CULTURE MEDIUM" HUMAN REPRODUCTION, IRL PRESS, OXFORD, GB, vol. 13, no. 6, June 1998 (1998-06), pages 1638-1644, XP009014102 ISSN: 0268-1161

## Description

### BACKGROUND OF THE INVENTION

### 1. Field of the Invention

The present invention is generally related to reproductive biology. More specifically, the present relates to improvements in methods of *in vitro* fertilization.

### 2. Description of Related Art

*In vitro* fertilization (IVF) of oocytes is a widely practiced medical technique used to overcome various forms of female and male infertility thereby providing for infertile couples. The standard IVF treatment is based on controlled ovarian hyperstimulation (COH) of female patients using exogenous hormones to induce the maturation of oocytes. The treatment is typically initiated by administering a gonadotropin releasing hormone (GnRH) agonist or antagonist to suppress the patient's own follicle stimulating hormone (FSH) and luteinizing hormone (LH). This is followed by injections of exogenous gonadotropins, e.g. FSH and/or LH, in order to ensure development of multiple preovulatory follicles. Just prior to ovulation, multiple *in vivo* matured oocytes are removed from the ovaries. The isolated mature oocytes are subsequently fertilized *in vitro* and cultured, typically for three to six days, before transferring the developed embryos back into the uterus at the 4-8 cell stage.

COH treatments are not successful in about one of five couples and are not recommended for a number of females, such as those females with polycistic ovary disease. Moreover, the exogenous hormone treatments used in COH treatments can over-stimulate follicular development and maturation of follicles. A subset of COH patients suffers from ovarian hyperstimulation syndrome (OHSS), which is a serious and potentially fatal condition. As a result, women undergoing COH must be closely monitored by daily ultrasound examinations of the ovaries and blood hormone measurements.

Due to the limitations of standard IVF treatments using COH, various alternative protocols have been suggested. One way to alleviate the risks, side effects, and economic disadvantages of COH protocols involves the retrieval of immature oocytes followed by maturation of the oocytes *in vitro*. In this approach, the female is without stimulation, or receives reduced stimulation, and the retrieved oocytes are subjected to hormonal treatment *in vitro. In vitro* maturation (IVM) of oocytes would allow a reduction or elimination of the amounts of exogenous hormones typically administered, thereby reducing the problems discussed.

Despite the success of IVF, there is a significant need for improved methods of infertility treatment. In particular, there is a significant need to develop methods of maturing oocytes *in vitro.* Methods of maturing oocytes in vitro are disclosed in Human Reproduction (1998) 13(6): 1638-1644.

### SUMMARY OF THE INVENTION

What the art needs are IVF protocols that reduce the occurrence of OHSS. The protocols should reduce or eliminate the amount of exogenous hormones administered to induce maturation of oocytes. The present invention satisfies these needs.

An oocyte may be matured *in vitro* by providing a composition comprising an immature oocyte. IL-17 may then be added to the composition, thereby inducing maturation of the oocyte. The mature oocyte may be used to produce an embryo by contacting the mature oocyte with sperm. The embryo may be implanted into the uterus of a female capable of carrying the embryo to term. The IL-17 may be any IL-17 type cytokine including, but not limited to, IL-17A, IL-17B, IL-17C, IL-17D, IL-17E or IL-17F.

### BRIEF DESCRIPTION OF THE DRAWINGS

Figure 1 shows the percentage of cumulus-oocyte complexes expanded following treatment with the indicated concentration of AS900048-6 (IL-17-6His).

Figure 2 shows the percentage of cumulus-oocyte complexes expanded following treatment with the indicated concentration of AS900269-1 (Met-IVKA-IL-17).

Figure 3 shows the percentage of cumulus-oocyte complexes expanded following treatment with the indicated concentration of IL-17B.

Figure 4 shows the percentage of cumulus-oocyte complexes expanded following treatment with the indicated concentration of IL-17D.

Figure 5 shows the percentage of cumulus-oocyte complexes expanded following treatment with the indicated concentration of IL-17F.

### DETAILED DESCRIPTION OF THE INVENTION

The present invention is related to the discovery that IL-17 induces the maturation of oocytes *in vitro.* By inducing the maturation of oocytes using IL-17, the amount of exogenous hormones administered in IVF treatment protocols may be reduced.

### 1. In Vitro Maturation

IL-17 may be used for the *in vitro* maturation of an oocyte.

### a. Oocytes

An immature oocyte may be retrieved from a female while the oocyte is at a stage of development including, but not limited to, early antral and antral follicles.

The immature oocyte may be retrieved from a female that has not undergone external hormonal therapy. Alternatively, the immature oocyte may be retrieved from a female that has undergone external hormonal therapy. The female may have been administered hormones including, but not limited to, GnRH, FSH, LH or hCG. The hormones may have been administered in combination or sequentially in any order.

The immature oocyte may be retrieved from the female by methods including, but not limited to, echography and aspiration. The immature oocyte may be cryopreserved after isolation and thawed at a later time for *in vitro* maturation.

### b. Maturation

The isolated immature oocyte may be incubated in a culture medium comprising an IL-17 cytokine. The culture medium may be any physiologically acceptable culture medium including, but not limited to, TCM 199, αMEM and Ham's F10. The culture medium may optionally further comprise one or more other factors including, but not limited to, FSH, hCG, estradiol, cysteamine, sodium pyruvate, glutamine, and antilogous heat-inactivated serum or follicular fluid.

The immature oocyte may be incubated in the culture medium at temperatures including, but not limited to, from about 37°C to about 39°C for a period of time including, but not limited to, about 6, 12, 18, 24, 30, 36, 42, 48, 54, 60, 66 or 72 hours. The oocyte may be incubated until maturation has occurred as evidenced by methods including, but not limited to, visual inspection under microscope of germinal vesicle break down (GVBD), cumulus expansion, metaphase II plate formation (MII), or polar body extrusion.

### c. Embryo Production

A mature oocyte may be incubated with sperm *in vitro* to produce a mammalian embryo using standard *in vitro* fertilization methods as described in Textbook of Assisted Reproductive Techniques Laboratory & Clinical Perspectives, edited by Gardner, et al., 2001 Martin Ldunetz Ltd., London. The embryo may be implanted into the uterus of a female capable of carrying the embryo to term.

### 2. IL-17

IL-17 is a family of structurally related cytokines. IL-17 cytokines exhibit pleiotropic biological activities on various types of cells, such as fibroblasts, endothelial cells, and epithelial cells. Representative examples of IL-17 cytokines include, but are not limited to, IL-17/IL17A, IL-17B, IL-17C, IL-17D, IL-17E and IL-17F. The IL-17 may also be an analog, derivative, fragment, homolog, variant, or combination thereof, of IL-17/IL-17A, IL-17B, IL-17C, IL-17D, IL-17E or IL-17F. The analog, derivative, fragment, homolog or variant may have at least 75%, 80%, 85%, 90% or 95% sequence identity with an IL-17. IL-17 cytokines may share a conserved C-terminal region but different N-terminal segments. The IL-17 cytokine may be a homodimer, which may be linked by a disulfide bond. The IL-17 may be human IL-17.

As used herein, the term "analog", when used in the context of IL-17, means a peptide or polypeptide comprising one or more non-standard amino acids or other structural variations from the conventional set of amino acids.

As used herein, the term "derivative", when used in the context of IL-17, means a peptide or polypeptide different other than in primary structure (amino acids and amino acid analogs). By way of illustration, derivatives may differ by being glycosylated, one form of post-translational modification. For example, peptides or polypeptides may exhibit glycosylation patterns due to expression in heterologous systems. If at least one biological activity is retained, then these peptides or polypeptides are derivatives according to the invention. Other derivatives include, but are not limited to, fusion peptides or fusion polypeptides having a covalently modified N- or C-terminus, PEGylated peptides or polypeptides, peptides or polypeptides associated with lipid moieties, alkylated peptides or polypeptides, peptides or polypeptides linked via an amino acid side-chain functional group to other peptides, polypeptides or chemicals, and additional modifications as would be understood in the art.

As used herein, the term "fragment", when used in the context of IL-17, means a peptide of from about 8 to about 50 amino acids in length. The fragment may be 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acids in length.

As used herein, the term "homolog", when used in the context of IL-17, means a peptide or polypeptide sharing a common evolutionary ancestor.

As used herein, the term "variant", when used in the context of IL-17, means a peptide or polypeptide that differs in amino acid sequence by the insertion, deletion, or conservative substitution of amino acids, but retain at least one biological activity. For purposes of the present invention, "biological activity" includes, but is not limited to, the ability to be bound by a specific antibody.

The present invention has multiple aspects, illustrated by the following non-limiting examples.

### EXAMPLES

### EXAMPLE 1

### Isolation Of Murine Cumulus-Oocyte Complex

PMSG (5 IU/female, Calbiochem 367222) was used to prime 7 to 8-week-old CD-1 female mice (35 total; Charles River). The mice were sacrificed 48 h later by progressive hypoxemia. Alcohol (70%) was applied to the abdominal region of the animals to clean the area and also to decrease contamination of samples with hair. A ventral incision was made to expose the abdominal cavity. The ovaries connected to oviducts were cut away from the uterine horn and the visceral adipose. The ovary/oviduct samples were placed in a 15 ml tubes (10 per tube, Corning 430052) containing 3 ml of L-15 medium (Gibco 11415-064) plus 10% fetal calf serum (FCS; Invitrogen 16000-044). The ovary/oviduct samples were maintained at 37°C.

The ovary/oviduct samples were then transferred to a Petri dish (Falcon 353004, 60x 15 mm). Under a stereomicroscope (Nikon SM2-800 with thermo-plate heating stage) using a pair of scissors needle (27 gauge) mounted in a 1 ml tuberculin syringe, the ovaries and oviduct were cleaned of the fatty pad and placed in a new Petri dish filled with 2-3 ml of fresh medium (L-15 + 10% FCS). The COCs were recovered by mechanical rupture of each ovary with needles and placed in a new Petri dish filled with 2-3 ml of fresh medium (L-15 + 10% FCS).

### EXAMPLE 2

### Effect Of IL-17 On The In Vitro Cumulus Expansion Of The Cumulus-Oocyte Complex

Cumulus-intact oocytes with homogeneous cytoplasm were selected from COCs prepared as described in Example 1 using a low-power (20-30 X) stereomicroscope and transferred using mouth glass pipets to 96-well plates (2/well) containing 90 µl culture media (αMEM (Gibco 32571-036) with 10% FCS and PenStrep-Antibiotics (Invitrogen 15140-122)) per well mineral oil. Before addition of the COCs to the 96-well plate, the medium in the plate was pre-equilibrated for a period of 1 h at 37°C in a humidified incubator with 5% CO₂ in air.

Different forms of IL-17 were added to each well in a volume of 10 µl so that the final volume in each well was 100 µl. Each plate contained 4 wells of a "Negative Control" (αMEM plus 10% FCS) and 4 wells of a "Positive Control" (αMEM plus 10% FCS plus EGF (5 ng/ml, Sigma E-9644)). Two plates, duplicates, were run per assay, providing 2 wells per test protein.
Proteins were diluted 1:5 in IVM medium (αMEM plus 10% FCS) before being added to the assay plates for a final dilution of 1:50 in the assay.

The plates containing the treated COCs were incubated for 18 h at 37°C in a humidified incubator with 5% CO₂ in air. Each COC was then visually inspected using a Nikon Inverted Microscope to identify the formation of a mucoid extracellular matrix by cumulus cells, which is an indicator of cumulus expansion. The percentage of cumulus expansion was defined as the number of expanded COCs in relation to the total COCs that were used in each treatment group. As shown in Table 1 below, each of the tested forms of IL-17 induced 80% expansion of COC in the primary assay.

**Table 1 - Primary COC Expansion Assay**

| Protein # | Protein | % Expanded |
|---|---|---|
| AS900231-1 | IL-17-ATT-6His | 80 |
| AS900231-2 | IL-17-ATT-6His | 80 |
| AS900269-1 | Met-IVKA-IL-17 | 80 |
| AS900048-4 | 1L-17-6His | 80 |

Each of the IL-17 proteins tested in the primary assay was then retested in a reconfirmation assay. The results from the reconfirmation assays in Table 2 indicate that each of the tested forms of IL-17 induced at least 80% expansion of COCs.

**Table 2 - Reconfirmation COC Expansion Assay**

| Protein # | Protein | % Expanded |
|---|---|---|
| AS900231-1 | IL-17-ATT-6His | 80 |
| AS900269-1 | Met-IVKA-IL-17 | 100 |
| AS900048-4 | IL-17-6His | 100 |

In both the primary assay and the reconfirmation assays, the COCs in the negative controls (no EGF) or the positive controls (plus EGF) wells were always 0% or 100% expanded, respectively (data not shown).

### EXAMPLE 3

### Dose-Response Analysis Of IL-17

Based on the results from the preliminary and reconfirmation assays described in Example 2, dose-response analysis was performed for AS900048-6 (IL-17-6His) and AS900269-1 (Met-IVKA-IL-17). Dose-response testing was performed similar to the method described in Example 2, except 3 wells with 4-5 COCs per well were assigned to each protein concentration. Dilutions of the test proteins were made depending on the concentration of the particular proteins, which were sometimes not diluted before being added to the assay, resulting in a final concentration of 1:10.

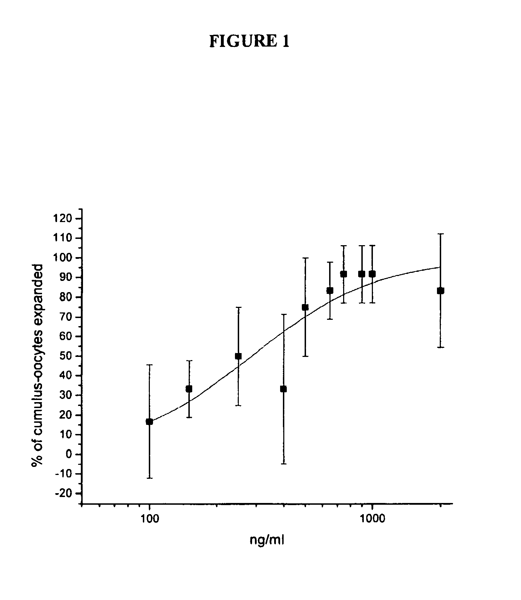

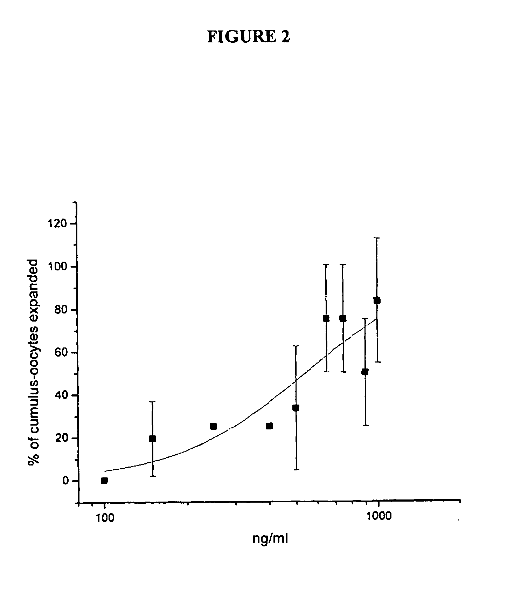

The results of the dose-response analysis of AS900048-6 (IL-17-6His) and AS900269-1 (Met-IVKA-IL-17) appear in Figure 1 and Figure 2, respectively. Analysis with Origin 7 SR3 v7.0475 (B475) indicates that the EC₅₀ for cumulus expansion with AS900048-6 (IL-17-6His) was 0.15 µg/ml whereas the EC₅₀ for cumulus expansion with AS900269-1 (Met-IVKA-IL-17) was 0.45 µg/ml.

### EXAMPLE 4

### Analysis Of IL-17 Cytokines

Different IL-17 cytokines were tested for the ability to induce *in vitro* cumulus expansion in the manner described in Example 2. As can be seen in Tables 3-8, IL-17B, IL-17C, IL-17D, IL-E and IL-F were also able to induce *in vitro* cumulus expansion. In addition, IL-17B, IL-17D and IL-17F had similar activity to IL-17A.

**Table 3 - IL-17A Expansion Assay**

| Protein | | Expanded Oocytes | Total Oocytes | % Expanded |
|---|---|---|---|---|
| IL-17A | 1 µg/ml | 3 | 3 | 100 |
| | | 3 | 3 | 100 |
| | | 3 | 3 | 100 |
| | 100 ng/ml | 2 | 3 | 66.7 |
| | | 3 | 3 | 100 |
| | | 0 3 0 | | |
| | 10 ng/ml | 0 | 3 | 0 |
| | | 0 3 0 | | |
| | | 0 3 0 | | |

**Table 4 - IL-17B Expansion Assay**

| Protein | | Expanded Oocytes | Total Oocytes | % Expanded |
|---|---|---|---|---|
| IL-17B | 1 µg/ml | 3 | 3 | 100 |
| | | 3 | 3 | 100 |
| | | 3 | 3 | 100 |
| | 100 ng/ml | 3 | 3 | 100 |
| | | 3 | 3 | 100 |
| | | 3 | 3 | 100 |
| | 10 ng/ml | 1 | 3 | 33.3 |
| | | 1 | 3 | 33.3 |
| | | 0 | 3 | 0 |

**Table 5 - IL-17C Expansion Assay**

| Protein | | Expanded Oocytes | Total Oocytes | % Expanded |
|---|---|---|---|---|
| IL-17C | 1 µg/ml | 2 | 3 | 66.7 |
| | | 1 | 3 | 33.3 |
| | | 0 | 3 | 0. |
| | 100 ng/ml | 2 | 3 | 66.7 |
| | | 1 | 3 | 33.3 |
| | | 1 | 3 | 33.3 |
| | 10 ng/ml | 0 | 3 | 0. |
| | | 1 | 3 | 33.3 |
| | | 0 | 3 | 0 |

**Table 6 - IL-17D Expansion Assay**

| Protein | | Expanded Oocytes | Total Oocytes | % Expanded |
|---|---|---|---|---|
| IL-17D | 1 µg/ml | 3 | 3 | 100 |
| | | 3 | 3 | 100 |
| | | 3 | 3 | 100 |
| | 100 ng/ml | 3 | 3 | 100 |
| | | 3 | 3 | 100 |
| | | 3 | 3 | 100 |
| | 10 ng/ml | 3 | 3 | 100 |
| | | 1 | 3 | 33.3 |
| | | 1 | 3 | 33.3 |

**Table 7 - IL-17E Expansion Assay**

| Protein | | Expanded Oocytes | Total Oocytes | % Expanded |
|---|---|---|---|---|
| IL-17E | 1 µg/ml | 0 | 3 | 0 |
| | | 1 | 2 | 50 |
| | | 1 | 3 | 33.3 |
| | 100 ng/ml | 1 | 3 | 33.3 |
| | | 1 | 3 | 33.3 |
| | | 0 | 3 | 0 |
| | 10 ng/ml | 1 | 3 | 33.3 |
| | | 1 | 3 | 33.3 |
| | | 0 | 3 | 0 |

**Table 8 - IL-17F Expansion Assay**

| Protein | | Expanded Oocytes | Total Oocytes | % Expanded |
|---|---|---|---|---|
| IL-17F | 1 □g/ml | 3 | 3 | 100 |
| | | 3 | 3 | 100 |
| | | 3 | 3 | 100 |
| | 100 ng/ml | 3 | 3 | 100 |
| | | 2 | 3 | 66.7 |
| | | 2 | 3 | 66.7 |
| | 10 ng/ml | 1 | 3 | 33.3 |
| | | 1 | 3 | 33.3 |
| | | 2 | 3 | 66.7 |

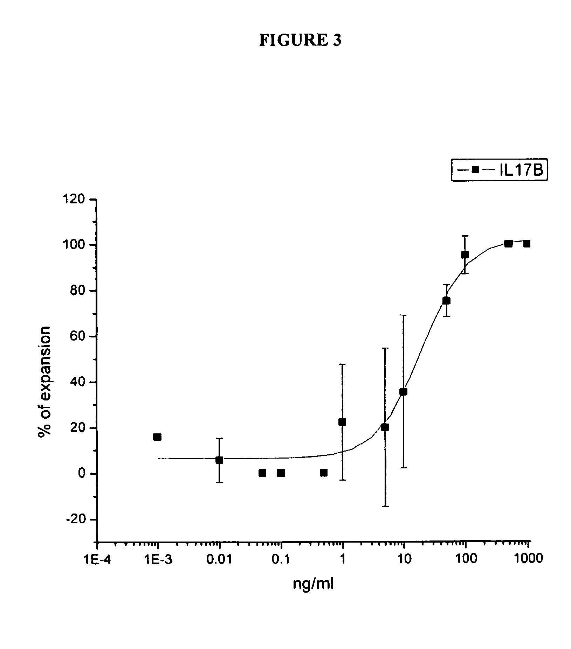

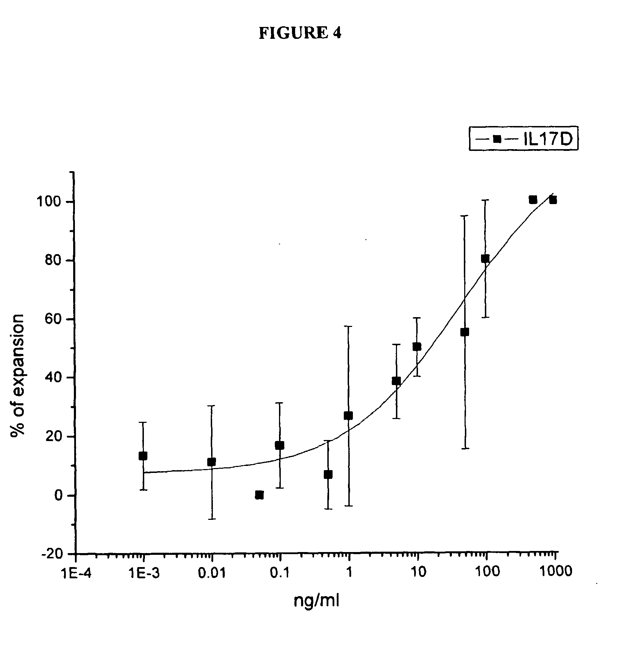

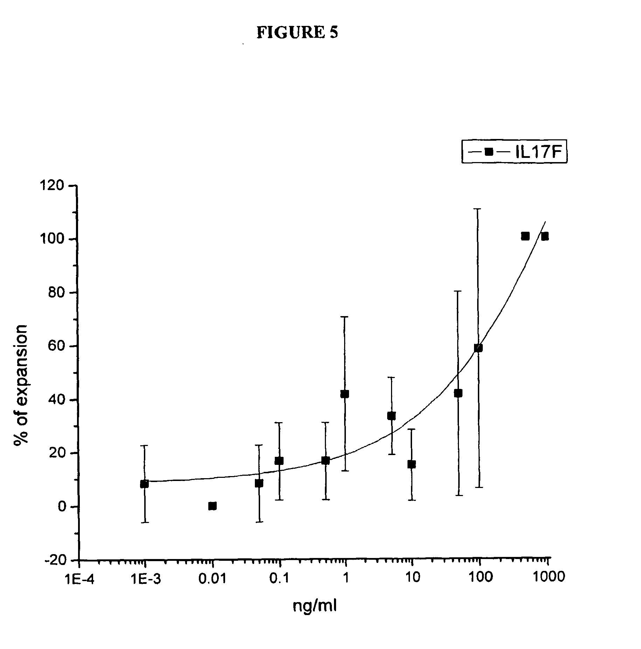

Based on these results, dose response analysis was performed on IL-17B, IL-17D and IL-17F in the manner described in Example 3. The results of the dose-response analysis appear in Figures 3-5. Analysis with Origin 7 SR3 v7.0475 (B475) indicates that the EC₅₀ for cumulus expansion with IL-17B, IL-17D and IL-17F is 12.3 ng/ml, 74 ng/ml and 50 ng/ml, respectively.

The description is not limited to the above embodiments.

## Claims

1. A method of maturing an oocyte in vitro comprising:
contacting an immature oocyte with IL-17.

2. The method of claim 1 wherein the IL-17 is selected from the group consisting of IL17/IL-17A, IL-17B, IL-17C, IL-17D, IL-17E and IL-17F.

3. The method of claim 1 wherein the IL-17 is IL17/IL-17A or a polypeptide at least 80% identical thereto.

4. The method of claim 1 wherein the immature oocyte is derived from a female that has not undergone external hormonal therapy.

5. The method of claim 1 wherein the immature oocyte is derived from a female that has undergone external hormonal therapy.

6. The method of claim 5 wherein the female was administered a hormone selected from the group consisting of GnRH, FSH, LH, hCG, and a combination thereof.

7. The method of claim 1 wherein the immature oocyte is provided in a culture medium comprising a factor selected from the group consisting of FSH, hCG, estrachol, cysteamine, sodium pyruvate, glutamine, autologous heat-inactivated serum, and follicular fluid.

8. A method of *in vitro* fertilization comprising producing a mature oocyte by a method according to claim 1 and treating the mature oocyte with sperm.

## Patentansprüche

1. Verfahren zur Reifung einer Oozyte in vitro, umfassend:
das Inkontaktbringen einer unreifen Oozyte mit IL-17.

2. Verfahren nach Anspruch 1, wobei das IL-17 ausgewählt ist aus der Gruppe bestehend aus IL17/IL-17A, IL-17B, IL-17C, IL-17D,IL-17E und IL-17F.

3. Verfahren nach Anspruch 1, wobei das IL-17 IL17/IL-17A oder ein zu mindestens 80 % damit identisches Polypeptid ist.

4. Verfahren nach Anspruch 1, wobei die unreife Oozyte von einem weiblichen Wesen erhalten wird, das keiner externen Hormontherapie unterzogen wurde.

5. Verfahren nach Anspruch 1, wobei die unreife Oozyte von einem weiblichen Wesen erhalten wird, das einer externen Hormontherapie unterzogen wurde.

6. Verfahren nach Anspruch 5, wobei dem weiblichen Wesen ein Hormon, ausgewählt aus der Gruppe bestehend aus GnRH, FSH, LH, hCG und einer Kombination davon, verabreicht wurde.

7. Verfahren nach Anspruch 1, wobei die unreife Oozyte in einem Kulturmedium bereitgestellt wird, das einen Faktor umfasst, der ausgewählt ist aus der Gruppe bestehend aus FSH, hCG, Estradiol, Cysteamin, Natriumpyruvat, Glutamin, autologem hitzeinaktiviertem Serum und Follikelflüssigkeit.

8. Verfahren zur *in vitro*-Fertilisation umfassend das Herstellen einer reifen Oozyte durch ein Verfahren nach Anspruch 1 und das Behandeln der reifen Oozyte mit Sperma.

## Revendications

1. Procédé de maturation d'un ovocyte *in vitro* comprenant :
le contact d'un ovocyte immature avec de l'IL-17.

2. Procédé selon la revendication 1, dans lequel l'IL-17 est choisi dans le groupe constitué par IL17/IL-17A, IL-17B, IL-17C, IL-17D, IL-17E et IL-17F.

3. Procédé selon la revendication 1, dans lequel l'IL-17 est IL-17/IL-17A ou un polypeptide qui lui est identique au moins à 80 %.

4. Procédé selon la revendication 1, dans lequel l'ovocyte immature provient d'une femelle qui n'a pas subi d'hormonothérapie externe.

5. Procédé selon la revendication 1, dans lequel l'ovocyte immature provient d'une femelle qui a subi une hormonothérapie externe.

6. Procédé selon la revendication 5, dans lequel on a administré à la femelle une hormone choisie dans le groupe constitué par GnRH, FSH, LH, hCG et une de leurs combinaisons.

7. Procédé selon la revendication 1, dans lequel l'ovocyte immature est fourni dans un milieu de culture comprenant un facteur choisi dans le groupe constitué par FSH, hCG, l'estrachol, la cystéamine, le pyruvate de sodium, la glutamine, un sérum autologue inactivé par la chaleur et un fluide folliculaire.

8. Procédé de fécondation *in vitro* comprenant la production d'un ovocyte mature par un procédé selon la revendication 1 et le traitement de l'ovocyte mature avec du sperme.
